# EUROPÄISCHE PATENTSCHRIFT

(11) **EP 2 473 476 B1**
(45) Veröffentlichungstag und Bekanntmachung des Hinweises auf die Patenterteilung: **14.01.2015**
(21) Anmeldenummer: 10745569.3
(22) Anmeldetag: 18.08.2010
(51) Int. Cl.: C07C 201/12, C07C 209/68, C07C 205/06, C07C 205/12, C07C 231/12, C07F 5/02, C07C 233/15

(54) **TETRAARYLBORAT VERFAHREN ZUR HERSTELLUNG VON SUBSTITUIERTEN BIPHENYLEN**
Tetraarylborate method for producing substituted biphenyls
Procédé tétraarylborate pour la fabrication de biphényles substitués

(30) Priorität: 31.08.2009 EP 09169039; 03.09.2009 US 239516 P
(43) Veröffentlichungstag der Anmeldung: 11.07.2012
(73) Patentinhaber: Bayer Intellectual Property GmbH, 40789 Monheim am Rhein (DE)
(72) Erfinder: MORADI, Wahed Ahmed, 40789 Monheim (DE); LUI, Norbert, 51519 Odenthal (DE); DOCKNER, Michael, 50674 Köln (DE); JAGUSCH, Thomas, 47559 Kranenburg (DE)
(74) Vertreter: BIP Patents
(86) Internationale Anmeldenummer: PCT/EP2010/005060
(87) Internationale Veröffentlichungsnummer: WO 2011/023324

(56) Entgegenhaltungen:
- JP-A- 2008 063 329
- US-A1- 2002 052 454
- US-A1- 2003 181 707
- US-B1- 6 339 161
- LU G ET AL: "Palladium charcoal-catalyzed, ligandless Suzuki reaction by using tetraarylborates in water" TETRAHEDRON LETTERS, ELSEVIER, AMSTERDAM, NL, Bd. 46, Nr. 24, 13. Juni 2005 (2005-06-13) , Seiten 4255-4259, XP025386182 ISSN: 0040-4039 [gefunden am 2005-06-13] in der Anmeldung erwähnt
- ANULEWICZ-OSTROWSKA R ET AL: "Synthesis of some halogenated tetraarylborates" TETRAHEDRON LETTERS, ELSEVIER, AMSTERDAM, NL, Bd. 44, Nr. 39, 22. September 2003 (2003-09-22), Seiten 7329-7331, XP004451294 ISSN: 0040-4039 in der Anmeldung erwähnt
- MIYAURA N ET AL: "PALLADIUM-CATALYZED CROSS-COUPLING REACTIONS OF ORGANOBORON COMPOUNDS" CHEMICAL REVIEWS, ACS,WASHINGTON, DC, US, Bd. 95, Nr. 7, 1. Januar 1995 (1995-01-01) , Seiten 2457-2483, XP000652239 ISSN: 0009-2665 in der Anmeldung erwähnt

## Beschreibung

Die vorliegende Erfindung betrifft ein Verfahren zur Herstellung von substituierten Biphenylen durch Umsetzung von Arylhalogeniden mit Tetraarylboraten in Gegenwart von Palladiumkatalysatoren.

Biarylverbindungen, insbesondere Biphenylverbindungen, haben technische Bedeutung als Feinchemikalien, Zwischenprodukte für Pharmazeutika, optische Aufheller und Agrochemikalien.

Eine häufig angewandte Methode zur Synthese von Biphenylen im Labormaßstab ist die Suzuki-Reaktion, bei der Iod- oder Bromaromaten und in Ausnahmefällen Chloraromaten mit Aryl-, Vinyl- oder Alkylboronsäure-Derivaten in Gegenwart von Palladium-Katalysatoren umgesetzt werden. Ubersichtsartikel, die diese Methodik beschreiben, findet man beispielsweise in N. Miyaura, A. Suzuki, Chem. Rev. 1995, 95, 2457 und Bellina, F. et al. Synthesis 2004, 2419. Eine Übersicht über die Verwendung von Trialkylphosphinliganden bei der Pd-katalysierten Umsetzung von Chloraromaten findet man in Littke, A.F. & Fu, G.C. Angew. Chem. 2002, 114. 4350.

Bei den im Stand der Technik beschriebenen Suzuki-Kupplungen werden häufig Arylboronsäuren als Kupplungspartner eingesetzt. Diese haben den Nachteil, dass mit jedem Äquivalent der eingesetzten Arylboronsäure lediglich ein Arylrest übertragen werden kann.

Bei all den im Stand der Technik beschriebenen Verfahren werden teure oder aufwändig herzustellende Palladiumkomplexe eingesetzt oder es ist nötig, zur Erzielung einer guten Ausbeute in Gegenwart eines Überschusses an Arylboronsäure zu arbeiten. Dies erhöht nicht nur die Kosten des Verfahrens durch den Verlust an wertvoller Arylboronsäure, sondern auch durch aufwändigere Reinigungs- und Isolierungsverfahren, die notwendig sind, um überschüssige Boronsäure sowie daraus entstandene Nebenprodukte wie deboronierte Aromaten und Homokupplungsprodukte abzutrennen.

WO 2006/092429 beschreibt die Umsetzung von aromatischen Borinsäuren mit Arylhalogeniden in wassrigen Lösungsmittelsystemen u.a. in Gegenwart von Trialkylphosphinen. Nachteilig erscheint bei diesem Verfahren, dass Borinsäuren jedoch nicht in allen Fällen leicht synthetisch zugänglich sind und dass die Reaktivität deutlich schlechter als die der entsprechenden Boronsäuren ist. Auch bei den Borinsäuren besteht weiterhin der Wunsch, die Anzahl der übertragbaren Arylreste zu steigern.

G. Lu et al. beschreiben in Tetrahedron Letters 2005, 46, 4255-4259 die Verwendung von Natriumtetraphenylboraten und Natriumtetratolylboraten als stabile und kommerziell zugängliche Boratquellen.

WO 2009/003650 lehrt, dass der Verlauf der Suzuki-Reaktion auch durch die Reaktivität der eingesetzten Boronsäure oder Borinsäure maßgeblich beeinflusst wird, wobei insbesondere durch elektronenziehende Substituenten desaktivierte Aromaten langsamer reagieren und Homokupplungsprodukte liefern können. Dieses Problem findet in der Literatur jedoch kaum Beachtung, da hier meist in einem großen Überschuss an Boronsäure gearbeitet wird und die Ausbeuten lediglich auf den Umsatz des Halogenaromaten bezogen werden.

Ein weiterer Nachteil der im Stand der Technik vorbeschriebenen Verfahren ist daher die konkurrierende Homokupplungsreaktion der Halogenaromaten unter Bildung von toxischen polyhalogenierten Biphenylen.

Elektronenarme Tetraarylborate gelten somit als zu wenig reaktiv und daher als in Suzuki Kopplungen ungeeignet.

Eine erste Aufgabe der vorliegenden Erfindung ist die Verbesserung der Raum-Zeit-Ausbeute der Suzuki Kupplung.

Eine weitere Aufgabe der vorliegenden Erfindung ist die Bereitstellung eines neuen Verfahrens zur Herstellung von elektrodenarmen Biphenylen, insbesondere von solchen, die mit mehreren (2, 3 oder 4) Halogenatomen substituiert sind, das die Nachteile der bekannten Verfahren nicht aufweist. Das Verfahren soll für die großtechnische Durchführung geeignet sein und elektronenarme Biphenyle in hoher Ausbeute und Reinheit bei optimaler Katalysatorproduktivität liefern.

Diese Aufgabe wird gelöst durch ein Verfahren zur Herstellung von substituierten Biphenylen der Formel (1) 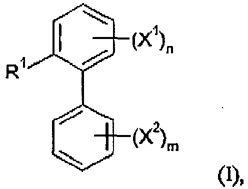 wobei
- X¹: ausgewählt ist aus Halogenatomen und linearen oder verzweigten C₁₋₁₂-Alkyl-Gruppen;
- X²: ausgewählt ist aus Halogenatomen;

- n: 0, 1 oder 2 ist;
- m: 1, 2, 3, 4 oder 5 ist;
- R¹: ausgewählt ist aus der Gruppe bestehend aus Amino- (NHR²), Nitro- (NO₂), Amid-Gruppen (R²-(CO)-NH-) oder Schiffschen Basen (R³R⁴C=N-),
- R², R³ und R⁴: ausgewählt sind aus linearen oder verzweigten C₁₋₁₂-Alkyl-Gruppen oder cyclischen
C₃₋₈-Alkyl-Gruppen, Benzyl-Gruppen, Benzoyl-Gruppen, Pyrazolyl-Gruppen der Formel (Ia), Pyridyl-Gruppen der Formel (Ib) 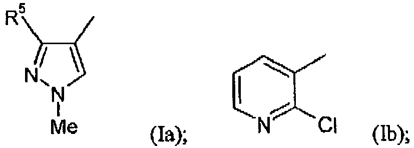
R⁵ eine lineare oder verzweigte C₁₋₁₂-Alkyl-Gruppe, oder eine C₁₋₆-Haloalkyl-Gruppe mit 1 bis 6 Halogenatomen ist;
durch Umsetzung von Arylhalogeniden der Formel (II) 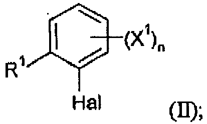 wobei
- Hal: ausgewählt ist aus Brom, Chlor und Iod,
- R¹, X¹ und n: den obigen Definitionen entsprechen;
in Gegenwart einer Base und eines Palladium Katalysators in einem Lösungsmittel,
mit Tetraarylboraten der Formel (III) 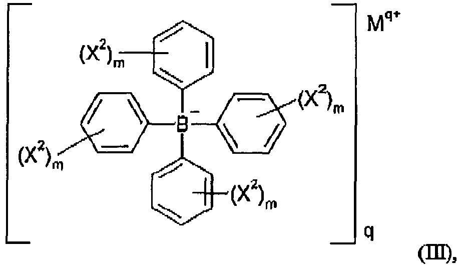 wobei
- X² und m: den obigen Definitionen entsprechen und
- M^{q+}: ein Kation ist, das ausgewählt ist aus Ammonium-(q=1), Alkalimetall- (q=1) und Erdalkalimetall-Kationen (q=2).

Das erfindungsgemäße Verfahren ermöglicht vorzugsweise die Kupplung von allen 4 Arylresten des Tetraarylborats (III) an die Arylhalogenide der Formel (II). Es verbessert somit die Raum-ZeitAusbeute der Suzuki Kupplung.

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, CICH₂, CF₃CCl₂.

Alkyl-Gruppen sind im Zusammenhang mit der vorliegenden Erfindung, soweit nicht abweichend definiert, lineare, oder verzweigte Kohlenwasserstoff-Gruppen, die optional ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₁-C₁₂-Alkyl umfasst den größten hierin definierten Bereich für eine Alkyl-Gruppe. Im Einzelnen umfasst diese Definition beispielsweise die Bedeutungen Methyl, Ethyl, n-, isoPropyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige Kohlenwasserstoff-Gruppen, die optional ein, zwei oder mehrere Heteroatome, die ausgewählt sind aus O, N, P und S aufweisen können. Außerdem können die erfindungsgemäßen Cycloalkyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₃-C₈-Cycloalkyl umfasst den größten hierin definierten Bereich für eine Cycloalkyl-Gruppe. Im Einzelnen umfasst diese Definition beispielsweise die Bedeutungen Cyclopropyl, Cyclobutyl, Cyclohexyl, Cycloheptyl und Cyclooctyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl-(-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazel-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus =R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl-(-(C=Q)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl-(-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

In einer bevorzugten Ausführungsform des erfindungsgemäßen Verfahren zur Herstellung von substituierten Biphenylen der Formel (I) sind die Substituenten wie folgt definiert:
- X¹: ist 5-Fluor;
- X²: ist 3/4-Chlor;
- n: ist 1;
- m: ist 2;
- R¹: ist ausgewählt aus der Gruppe bestehend aus Amino- (NH₂), Nitro- (NO₂), Amid-Gruppen (R²-(CO)-NH-) oder Schiffschen Basen (R³R⁴C=N-),
- R², R³ und R⁴: sind ausgewählt aus linearen oder verzweigten C₁₋₁₂-Alkyl-Gruppen oder cyclischen C₃₋₈-Alkyl-Gruppen.

In einer weiteren bevorzugten Ausführungsform des erfindungsgemäßen Verfahrens zur Herstellung von substituierten Biphenylen der Formel (1) sind die Substituenten wie folgt definiert:
- X¹: ist Wasserstoff;
- X²: ist 3,4,5-Fluor;
- n: ist 1;
- m: ist 3;
- R¹: ist ausgewählt aus der Gruppe bestehend aus Amino- (NH2), Nitro- (NO2), Amid-Gruppen (R₂-(CO)-NH-) oder Schiffschen Basen (R³R⁴C=N-) ist,
- R², R³ und R⁴: sind ausgewählt aus linearen oder verzweigten C₁₋₁₂-Alkyl-Gruppen oder cyclischen C₃₋₈-Alkyl-Gruppen.

Die Arylhalogenide der Formel (II) sind im Zusammenhang mit der vorliegenden Erfindung Chlor-, Brom- oder Iodaromaten.

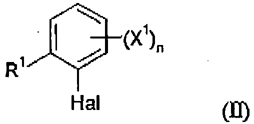

In Formel (II) ist
- X¹: ausgewählt aus Halogenatomen und linearen oder verzweigten C₁₋₁₂-Alkyl-Gruppen, vorzugsweise 5-Fluor;
- n: 0, 1 oder 2, vorzugsweise 0 oder 1;
- R¹: ausgewählt aus der Gruppe bestehend aus Amino- (NHR²), Nitro- (NO₂), Amid-Gruppen (R²-(CO)-NH-) oder Schiffschen Basen (R³R⁴C=N-), vorzugsweise aus Amino- und Nitro-Gruppen;
- R², R³ und R⁴: ausgewählt sind aus linearen oder verzweigten C₁₋₁₂-Alkyl-Gruppen oder cyclischen C₃₋₈-Alkyl-Gruppen, Benzyl-Gruppen, Benzoyl-Gruppen, Pyrazolyl-Gruppen der Formel (Ia), Pyridyl-Gruppen der Formel (Ib),

In einer bevorzugten Ausführungsform sind die Arylhalogenide der Formel (II) ausgewählt aus Anilinen (R¹=Amino), besonders bevorzugt sind 2-Bromanilin und 2-Brom-4-fluoranilin.

In einer alternativen bevorzugten Ausführungsform der Erfindung sind die Arylhalogenide der Formel (II) ausgewählt aus Acetaniliden (R¹= CH₃-(CO)-NH-), besonders bevorzugt sind 2-Bromacetanilid und 2-Brom-4-fluoracetanilid.

In einer weiteren bevorzugten Ausführungsform der Erfindung sind die Arylhalogenide der Formel (II) ausgewählt aus der Gruppe bestehend aus N-(2-Brom-4-fluorophenyl)acetamid, N-(2-Chlor-4-fluorphenyl)acetamid, N-(2-Bromphenyl)acetamid, N-(2-Chlorophenyl)acetamid, N-(2-Chlorphenyl)-3-oxobutanamid, N-(2-Bromophenyl)-3-oxobutanamid, N-(2-Chlor-4-fluorphenyl)-3-oxo-butanamid, N-(2-Brom-4-fluorphenyl)-3-oxobutanamid, 2-Brom-N-(propan-2-yliden)anilin, 2-Chlor-N-(propan-2-yliden)anilin, 2-Brom-4-fluor-N-(propan-2-yliden)anilin, 2-chloro-4-fluoro-N-(propan-2-ylidene)anilin.

In einer weiteren bevorzugten Ausführungsform der Erfindung sind die Arylhalogenide der Formel (II) ausgewählt aus Pyrazolyl- oder Pyridyl-Aniliden (R¹ = R²-(CO)-NH-), die Pyrazolyl-Gruppen der Formel (Ia) mit R⁵ = CHF₂ oder Pyridyl-Gruppen der Formel (Ib) enthalten.

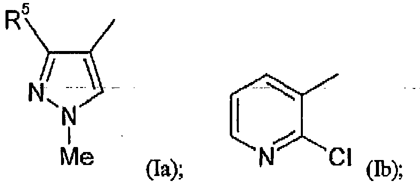

Besonders bevorzugt sind N-(2-Bromphenyl)-3-(difluormethyl)-1-methyl-1H-pyrazol-4-carboxamid, N-(2-Bromo-4-fluorphenyl)-3-(difluormethyl)-1-methyl-1H-pyrazol-4-carboxamid und N-(2-Bromphenyl)-2-chlornicotinamid.

Die erfindungsgemäßen Tetraarylborate sind Verbindungen der Formel (III) 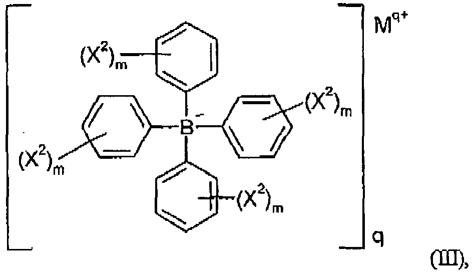 wobei
- X²: ausgewählt ist aus Halogenatomen, besonders bevorzugt ist X² Chlor oder Fluor;
- m: 1,2,3,4 oder 5 ist, vorzugsweise 1, 2 oder 3, besonders bevorzugt 2 oder 3.
- M: ein Kation ist, welches beispielsweise ausgewählt ist aus der Gruppe bestehend aus Ammonium (q=1), Alkalimetallen, z.B. Lithium, Natrium und Kalium (q=1), Erdalkalimetallen, z.B. Magnesium, Calcium oder Barium (q=2), oder komplexen Erdalkalimetallhalogeniden, wie z.B. [MgCl]⁺, [MgBr]⁺, [CaBr]⁺, [CaCl]⁺(q=1).

In einer bevorzugten Ausführungsform der Erfindung ist das Tetraarylborat der Formel (III) ausgewählt aus der Gruppe bestehend aus Natriumtetrakis(3,4-dichlorphenyl)borat, Kaliumtetrakis(3,4 dichlorphenyl)borat, Natriumtetrakis(4-chlorphenyl)borat, Kaliumtetrakis(4-chlor-phenyl)borat, Natriumtetrakis(3,4,5-trifluorphenyl)borat, Kaliumtetrakis(3,4,5-trifluorphenyl)borat. Im Zusammenhang mit der vorliegenden Erfindung besonders bevorzugte Tetraarylborate sind Natriumtetrakis(3,4-dichlorphenyl)borat, Natriumtetrakis(3,4,5-trifluorphenyl)borat, Natrium-tetra-kis(4-chlorphenyl)borat.

Die Tetraarylborate können beispielsweise gemäß der Synthesevorschrift in J. Serwatoski et al. Tetrahedron Letters 2003, 44, 7329 hergestellt werden.

Die Kupplung der Tetraarylborate der Formel (III) mit den Arylhalogeniden der Formel (II) findet vorzugsweise in Gegenwart mindestens eines Lösungsmittels statt, welches beispielsweise ausgewählt ist aus der Gruppe bestehend aus Wasser, aliphatischen Ethern, ggfs. halogenierten aromatischen oder aliphatischen Kohlenwasserstoffen, Alkoholen, Estern, aromatischen oder aliphatischen Nitrilen und dipolar aprotischen Lösungsmitteln, wie Dialkylsulfoxiden, N,N-Dialkylamiden aliphatischer Carbonsäuren oder alkylierten Lactamen.

Besonders bevorzugt sind Lösungsmittel, die ausgewählt sind aus der Gruppe bestehend aus THF, Dioxan, Diethylether, Diglyme, Methyl-tert-butylether (MTBE), tert-Amyl-methylether (TAME), Dimethylether (DME), 2-Methyl-THF, Acetonitril, Butyronitril, Toluol, Xylole, Mesitylen, Anisol, Ethylacetat, Isopropylacetat, Methanol, Ethanol, Propanol, Butanol, Ethylenglycol, Ethylencarbonat, Propylencarbonat, N,N-Dimethylacetamid, N,N-Dimethylformamid, N-Methylpyrrolidon, Wasser und Gemischen dieser.

Ganz besonders bevorzugt sind Mischungen aus Toluol, THF oder Dioxan und Wasser.

Es wurde zudem beobachtet, dass der Zusatz geringer Mengen (bis zu 20% des Lösungsmittels) Wasser zu den organischen Lösungsmitteln zu einer weitgehenden Unterdrückung der konkurrierenden Homokupplungsreaktion beiträgt.

Aufgrund der Löslichkeiten der Edukte und der entstehenden Produkte kann jedoch im Allgemeinen nicht gänzlich auf die Anwesenheit eines organischen (unpolaren) Lösungsmittels verzichtet werden. Daher werden die organischen Lösungsmittel vorzugsweise als Cosolvenzien eingesetzt.

Die erfindungsgemäßen Lösungsmittelgemische können zwischen 0,1 und 95 Volumen-% und vorzugsweise zwischen 1 und 60 Volumen-% Wasser, bezogen auf die Mischung aus Wasser und dem organischen Lösungsmittel, enthalten.

Da bei der Reaktion eine Säure gebildet wird, ist es vorteilhaft, die entstehende Säure durch Zusatz einer Base abzufangen. Die Base kann entweder von Beginn an vorhanden sein oder während der Reaktion kontinuierlich zudosiert werden (semi-batch Verfahren).

Gemäß der vorliegenden Erfindung geeignete Basen sind beispielsweise primäre, sekundäre und tertiäre Amine wie beispielsweise Alkylamine, Dialkylamine, Trialkylamine, die alicyclisch oder offenkettig sein können; Alkali- und Erdalkalisalze aliphatischer und/oder aromatischer Carbonsäuren, wie Acetate, Propionate oder Benzoate; Alkali- und Erdalkali-Carbonate, Hydrogencarbonate, Phosphate, Hydrogenphosphate und/oder Hydroxide; sowie Metallalkoxide, insbesondere Alkali- oder Erdalkalialkoxide, wie beispielsweise Natriummethanolat, Kaliummethanolat, Natriumethanolat, Magnesiummethanolat, Calciumethanolat, Natrium-tert. butylat, Kalium-tert.-butylat oder Alkali-isoamylate. Vorzugsweise ist die Base ein Carbonat, Hydroxid oder Phosphat von Lithium, Natrium, Kalium, Calcium, Magnesium oder Cäsium. Besonders bevorzugt sind NaOH, KOH, Pottasche und Soda.

Die Suzuki-Kupplung verläuft in Gegenwart von Palladiumkatalysatoren. Grundsätzlich können alle im Stand der Technik im Zusammenhang mit Suzuki Kupplungen beschriebenen Palladiumkatalysatoren verwendet werden.

Vorzugsweise werden Palladiumkatalysatoren eingesetzt, die ausgewählt sind aus den folgenden Gruppen (a) bis (c):
a) Palladium-Komplexe umfassend Palladium in der Oxidationssufe Null und Phosphinliganden der allgemeinen Formel PR'₃, wobei R' unabhängig voneinander ausgewählt ist aus der Gruppe bestehend aus C₁₋₆-Alkyl-, C₃₋₅-Cycloalkyl und C₆₋₁₂-Aryl oder Phosphinoferrocenliganden,
b) Palladiumsalze in Gegenwart von Phosphinliganden der allgemeinen Formel PR'₃, wobei R' unabhängig voneinander ausgewählt ist aus der Gruppe bestehend aus C₁₋₆-Alkyl-, C₃₋₅-Cycloalkyl und C₆₋₁₂-Aryl oder in Gegenwart von Phosphinoferrocenliganden;
c) Palladiummetall, das ggf. auf einem Träger aufgebracht ist, wobei optional Phosphinliganden der allgemeinen Formel PR'₃, wobei R' unabhängig voneinander ausgewählt ist aus der Gruppe bestehend aus C₁₋₆-Alkyl-, C₃₋₅-Cycloalkyl und C₆₋₁₂-Aryl oder Phosphinoferrocenliganden zugegeben werden können.

In einer bevorzugten Ausführungsform der Erfindung der Palladiumkatalysator der Kategorie (a) ausgewählt ist aus der Gruppe bestehend aus Tetrakis(triphenylphosphin)palladium, Tetrakis(tri-tert-butylphosphin)palladium, Adamantan-1-yl(adamantan-2-yl)butylphosphinpalladium, Biphenyl-2-yl(di-tert-butyl)phosphinpalladium oder 1,1-Bis-(di-tert-butylphosphino)ferrocenpalladium, Pentaphenyl(di-tert-butylphosphino)ferrocenpalladium, 1,3-Bis-(di-tert-butylphosphino-methylen)phenylpalladium.

In einer weiteren bevorzugten Ausführungsform der Erfindung ist der Palladium Katalysator der Kategorie (b) ausgewählt ist aus der Gruppe bestehend aus Palladiumchlorid, Palladiumacetat oder Bisacetonitrilpalladiumchlorid, Palladium(II)dibenzylidenaceton, Bisacetylacetonatpalladium.

Die eingesetzten Palladiumkatalysatoren werden in der Regel *in situ* aus mindestens einem Palladium(II)salz oder einer Palladium(0)-Verbindung und den entsprechenden Phosphinliganden erzeugt. Sie können jedoch auch als Palladium(0)-Verbindung direkt eingesetzt werden, ohne dass dadurch die anfängliche katalytische Aktivität gemindert wird.

Geeignete Palladiumquellen sind beispielsweise ausgewählt aus der Gruppe bestehend aus Palladiumtrifluoracetat, Palladiumfluoracetylacetonat, Palladiumchlorid, Palladiumacetat, Pd(OCOCH₂CH₃)₂, Pd(OH)₂, PdBr₂, Bisacetylacetonatpalladium, Pd(NO₃)₂, Palladiumdibenzylidenaceton, Pd₂dba₃, (dba = Dibenzylidenaceton), Pd(CH₃CN)₂Cl₂, Pd(PhCN)₂Cl₂, Li[PdCl₄], Pd/C oder Palladiumnanopartikeln.

Gemäß der vorliegenden Erfindung werden zur Kupplung von elektrodenarmen Boraten, vorzugsweise elektronenreiche und/oder sterisch gehinderte Phosphine in Kombination mit der Palladium(0) Quelle eingesetzt.

Beispiele dafür sind im Alkyl-Teil verzweigte Methyl-di(C₃₋₈-alkyl)phosphin- oder Tri(C₃₋₈-alkyl)phosphinliganden oder deren Salze, besonders bevorzugt von Methyl-di(tert-butyl)phosphin und Tri(tert-butyl)phosphin. Weitere Beispiele sind 1,3-Bis-(di-tert-butylphosphinomethylen)-phenyl, Adamantan-1-yl(adamantan-2-yl)butylphosphin, Biphenyl-2-yl(di-tert-butyl)phosphin, 1,1-Bis-(di-tert-butylphosphino)-ferrocen, 1,3-Bis-(di-tert-butylphosphinomethylen)phenyl, Pentaphenyl- (di-tert-butylphosphino)ferrocen.

Die Kombination von Tri(tert-butyl)phosphin mit Pd₂dba₃ hat sich als besonders vorteilhaft im Hinblick auf Reaktivität und Bildung von Homokupplungsprodukten erwiesen.

Das Trialkylphosphin kann auch als Trialkylphosphonium-Salz wie z.B. als Tetrafluoroborat (Org. Lett. 2001, 3, 4295), Perchlorat oder Hydrogensulfat eingesetzt und hieraus *in situ* durch Base freigesetzt werden.

Das molare Verhältnis von Palladium zum Phosphinliganden oder Phosphinoferrocenylliganden sollte zwischen 4.1 und 1:50 liegen, und liegt Vorzugsweise zwischen 1 : 1 und 1 : 5, besonders bevorzugt zwischen 1 : 1 und 1 : 2.

In einer bevorzugten Ausführungsform der Erfindung enthält der Palladium Katalysator der Kategorie (b) 6 bis 60 Äquivalente Triphenylphosphin oder Tri-tert-butylphosphin pro Äquivalent Palladiumsalz.

Erfindungsgemäß kann aber auch direkt Pd[P(tert-But)₃]₂ verwendet werden, dessen Herstellung in JACS 1976, 98, 5850; JACS 1977, 99, 2134 und in JACS 2001, 123, 2719 beschrieben ist.

Bei der Durchführung der Reaktion kann das Katalysatorsystem (Pd + Ligand) zusammen oder getrennt entweder bei Raumtemperatur oder in der Wärme zugegeben werden. Man kann das System kurz vor der Durchführung separat durch Zusammengeben eine Pd-Salzes und des Liganden herstellen oder in kristalliner Form käuflich erwerben. Man kann auch direkt in den Ansatz erst den Liganden und anschließend das Palladiumsalz hinzufügen (*in situ*-Verfahren).

Gemäß der vorliegenden Erfindung werden die Arylhalogenide der Formel (II) und die Tetraarylborate der Formel (III) im Verhältnis 4:1, vorzugsweise im Verhältnis 3:1 (II:III) eingesetzt. Alternativ kann jedoch auch eine der beiden Komponenten (II oder III), vorzugsweise das Tetraarylborat (III) im Überschuss eingesetzt werden. Es ist auch möglich, die Reaktion dosierkontrolliert durchzuführen, wobei eine der beiden Reaktionskomponenten während der Reaktion langsam zudosiert wird. Bevorzugt wird hierzu eine Lösung des Tetraarylborats (III) zudosiert, während das Arylhalogenid (II), der Katalysator und ggf. die Base vorgelegt werden. Es wurde beobachtet, dass diese erfindungsgemäße Vorgehensweise die Bildung von polychlorierten Biphenylen reduziert, die Produkte der Homokupplung sind.

Die Umsetzung wird im allgemeinen bei einer Temperatur von 20 bis 200°C, vorzugsweise von 40 bis 100°C, besonders bevorzugt von 60 bis 90°C, sowie bei einem Druck bis zu 100 bar, vorzugsweise bei einem Druck zwischen Normaldruck und 40 bar, durchgeführt.

Die Reaktion erfolgt vorzugsweise unter Ausschluss von Luftsauerstoff unter Schutzgasatmosphäre, wie z.B. unter Argon- oder Stickstoffatmosphäre.

Aufgrund der Katalysatoraktivitäten und -stabilitäten ist es mit dem erfindungsgemäßen Verfahren möglich, extrem kleine Mengen an Katalysator zu verwenden, so dass die Katalysatorkosten im Vergleich zu den bekannten Suzuki-Reaktionen für den entsprechenden Prozess nicht limitierend sind.

Bei dem erfindungsgemäßen Verfahren werden 0,001 bis 10,0 mol%, vorzugsweise 0,005 bis 3,0 mol%, besonders bevorzugt 0,01 bis 1,0 mol% des Palladium Katalysators - bezogen auf das Arylhalogenid der Formel (II) - eingesetzt

Aufgrund der geringen Katalysatormengen kann der Katalysator in den meisten Fällen im Endprodukt verbleiben. Alternativ kann jedoch auch eine Aufreinigung der erhaltenen Biaryle durch Filtration z.B. über Celite erfolgen.

Die nachstehenden Beispiele dienen der Erläuterung des erfindungsmäßen Verfahrens, ohne es darauf zu beschränkten:

### Synthesebeispiele:

### Beispiel 1: Kupplung von N-(2-Brom-4-fluorphenyl)acetamid mit Natriumtetrakis(3,4-dichlorphenyl)borat in Gegenwart von Bipbenyl-2-yl(di-tert-butyl)phosphin

96 mg [414 µmol] ***N*-(2-Brom-4-fluorphenyl)acetamid,** 71,6 mg [116 µmol] Natriumtetrakis(3,4-dichlorphenyl)borat und 91,3 mg [861 µmol] Natriumcarbonat werden unter Ausschluss von Sauerstoff in 0,8 ml Toluol und 0,2 ml Wasser vorgelegt. Zu dieser Mischung werden 4,76 mg [16 µmol] Biphenyl-2-yl(di-tert-butyl)phosphin (Maßlösung in Toluol) und 9,4 mg [10 µmol] Pd₂dba₃ zugesetzt, Die Reaktionsmischung wird 19 Stunden bei 82 °C gerührt. Nach Beendigung der Umsetzung (HPLC-Kontrolle) wird die Reaktionsmischung auf RT abgekühlt, 2 ml Acetonitril zugefügt und anschließend über einen Nylon-Filter (Porengröße 0,45 µm) filtriert. Die HPLC-Analyse der Mischung ergab folgendes Verhältnis: *N*-(3',4'-Dichlor-5-fluorbiphenyl-2-yl)acetamid : 3,3',4,4'-Tetrachlorbiphenyl : *N*-(2-Brom-4-fluorphenyl)acetamid = 99,5 : 0,5 : 0.

### Beispiel 2: Kupplung von N-(2-Brom-4-fluorphenyl)acetamid mit Natriumtetrakis(3,4-dichlorphenyl)borat in Gegenwart von Tri(tert-butyl)phosphin

95,7 mg [412 µmol] *N*-(2-Brom-4-fluorphenyl)acetamid, 69,2 mg [112 µmol] Natriumtetrakis(3,4-dichlorphenyl)borat und 94,3 mg [890 µmol] Natriumcarbonat werden unter Ausschluss von Sauerstoff in 0,8 ml Toluol und 0,2 ml Wasser vorgelegt. Zu dieser Mischung werden 3,16 mg [16 µmol]Tri(tert-butylphosphin (Maßlösung in Toluol) und 16,4 mg [18 µmol] Pd₂dba₃ zugesetzt. Die Reaktionsmischung wird 19 Stunden bei 82 °C gerührt. Nach Beendigung der Umsetzung (HPLC-Kontrolle) wird die Reaktionsmischung auf RT abgekühlt, 2 ml Acetonitril zugefügt und anschließend über einen Nylon-Filter (Porengröße 0,45 µm) filtriert. Die HPLC-Analyse der Mischung ergab folgendes Verhältnis: *N*-(3',4'-Dichlor-5-fluorbiphenyl-2-yl)acetamid : 3,3',4,4'-Tetrachlorbiphenyl : *N*-(2-brom-4-fluor-phenyl)acetamid = 96,7 : 1,15 : 2,15.

### Beispiel 3: Kupplung von N-(2-Brom-4-fluorphenyl)acetamid mit Natriumtetrakis(3,4-dichlorphenyl)borat in Gegenwart von 1,1-Bis(di-tert-butylphosphino)ferrocen

102,1 mg [440 µmol] *N*-(2-Brom-4-fluorphenyl)acetamid, 65,3 mg [106 µmol] Natriumtetrakis-(3,4-dichlorphenyl)borat und 100,5 mg [948 µmol] Natriumcarbonat werden unter Ausschluss von Sauerstoff in 0,8 ml Toluol und 0,2 ml Wasser vorgelegt. Zu dieser Mischung werden 6,89 mg [14 µmol] 1,1-Bis(di-tert-butylphosphino)ferrocen (Maßlösung in Toluol) und 10,1 mg [11 µmol] Pd₂dba₃ zugesetzt. Die Reaktionsmischung wird 19 Stunden bei 82 °C geführt. Nach Beendigung der Umsetzung (HPLC-Kontrolle) wird die Reaktionsmischung auf RT abgekühlt, 2 ml Acetonitril zugefügt und anschließend über einen Nylon-Filter (Porengröße 0,45 µm) filtriert. Die HPLC-Analyse der Mischung ergab folgendes Verhältnis: *N*-(3',4'-Dichlor-5-fluorbiphenyl-2-yl)acetamid : 3,3',4,4'-Tetrachlorbiphenyl : *N*-(2-Brom-4-fluor-phenyl)acetamid = 94,21 : 0,64 : 5,15.

### Beispiel 4: Kupplung von N-(2-Brom-4-fluorphenyl)acetamid mit Natriumtetrakis(3,4-dichlorphenyl)borat in Gegenwart von 1,3-Bis(di-tert-butylphosphinomethylen)phenyl

96 mg [413 µmol] *N*-(2-Brom-4-fluorphenyl)acetamid, 85,1 mg [0,137 mmol] Natriumtetrakis-(3,4-dichlorphenyl)borat und 92,6 mg [873 µmol] Natriumcarbonat werden unter Ausschluss von Sauerstoff in 0,8 ml Toluol und 0,2 ml Wasser vorgelegt. Zu dieser Mischung werden 6,68 mg [16,9 µmol] 1,3-Bis(di-tert-butylphosphosphinomethylen)phenyl (Maßlösung in Toluol) und 8,71 mg [9,5 µmol] Pd₂dba₃ zugesetzt. Die Reaktionsmischung wird 19 Stunden bei 82 °C gerührt. Nach Beendigung der Umsetzung (HPLC-Kontrolle) wird die Reaktionsmischung auf RT abgekühlt, 2 ml Acetonitril zugefügt und anschließend über einen Nylon-Filter (Porengröße 0,45 µm) filtriert. Die HPLC-Analyse der Mischung ergab folgendes Verhältnis: *N*-(3,4'-Dichlor-5-fluorbiphenyl-2-yl)acetamid : 3,3',4.4'-Tetrachlorbiphenyl; *N*-(2-Brom-4-fluorphenyl)acetamid = 99,3 : 0,70: 0.

### Beispiel 5: Kupplung von N-(2-Brom-4-fluorphenyl)atetaniid mit Natriumtetrakis(3,4-dichlorphenyl)borat in Gegenwart von 1,2,3,4,5-Pentaphenyl-1'(di-tert-butylphosphino)-ferrocen

104,8 mg [452 µmol] *N*-(2-Brom-4-fluorphenyl)acetamid, 74,2 mg [120 µmol] Natriumtetrakis-(3,4-dichlorphenyl)borat und 90,9 mg [857 µmol] Natriumcarbonat werden unter Ausschluss von Sauerstoff in 0,8 ml Toluol und 0,2 ml Wasser vorgelegt. Zu dieser Mischung werden 13,79 mg [19,4 µmol] 1,2,3,4,5-Pentaphenyl-1'-(di-tert-butylphosphino)ferrocen (Maßlösung in Toluol) und 8,60 mg [9,4 mmol] Pd₂dba₃ zugesetzt. Die Reaktionsmischung wird 19 Stunden bei 82 °C geführt. Nach Beendigung der Umsetzung (HPLC-Kontrolle) wird die Reaktionsmischung auf RT abgekühlt, 2 ml Acetonitril zugefügt und anschließend über einen Nylon-Filter (Porengröße 0,45 µm) filtriert. Die HPLC-Analyse der Mischung ergab folgendes Verhältnis: *N*-(3',4'-Dichlor-5-fluorbiphenyl-2-yl)acetamid : 3,3',4,4'-Tetrachlorbiphenyl : *N*-(2-Brom-4-fluorphenyl)acetamid = 97,96 : 1,31 : 0,73.

### Beispiel 6: Kupplung von N-(2-Brom-4-fluorphenyl)acetamid mit Natriumtetrakis(3,4,5-trifluorphenyl)borat in Gegenwart von Biphenyl-2-yl(di-tert-butyl)phosphin

104,1 mg [449 µmol] *N*-(2-Brom-4-fluorphenyl)acetamid, 59,0 mg [106 µmol] Natriumtetrakis-(3,4,5-trifluorphenyl)borat und 111,3 mg [1050 µmol] Natriumcarbonat werden unter Ausschluss von Sauerstoff in 0,8 ml Toluol und 0,2 ml Wasser vorgelegt. Zu dieser Mischung werden 4,55 mg [15.3 µmol] Biphenyl-2-yl(di-tert-butyl)phosphin (Maßlösung in Toluol) und 10,6 mg [11,7 µmol] Pd₂dba₃ zugesetzt. Die Reaktionsmischung wird 65 Stunden bei 82 °C gerührt. Nach Beendigung der Umsetzung (HPLC-Kontrolle) wird die Reaktionsmischung auf RT abgekühlt, 2 ml Acetonitril zugefügt und anschließend über einen Nylon-Filter (Porengröße 0,45 µm) filtriert. Die HPLC-Analyse der Mischung ergab folgendes Verhältnis: *N*-(3'.4',5'-Trifluor-5-fluorbiphenyl)acetamid : 3,3',4,4',5,5'-Hexafluorobiphenyl : *N*-(2-Brom-4-fluorphenyl)acetamid = 90,62 : 1,48 : 7,90.

### Beispiel 7: Kupplung von N-(2-Brom-4-fluorphenyl)acetamid mit Natriumtetrakis(3,4,5-trifluorphenyl)borat in Gegenwart von Tri(tert-butyl)phosphin

102,9 mg [443 µmol] *N*-(2-Brom-4-fluorphenyl)acetamid, 70,7 mg [127 µmol] Natriumtetrakis-(3,4,5-trifluorphenyl)berat und 100,7 mg [950 µmol] Natriumcarbonat werden unter Ausschluss von Sauerstoff in 0,8 ml Toluol und 0,2 ml Wasser vorgelegt. Zu dieser Mischung werden 3,50 mg [17,3 µmol] Tri(tert-butyl)phosphin (Maßlösung in Toluol) und 6,90 mg [7,5 mmol] Pd₂dba₃ zugesetzt. Die Reaktionsmischung wird 65 Stunden bei 82 °C gerührt. Nach Beendigung der Umsetzung (HPLC-Kontrolle) wird die Reaktionsmischung auf RT abgekühlt, 2 ml Acetonitril zugefügt und anschließend über einen Nylon-Filter (Porengröße 0,45 µm) filtriert. Die HPLC-Analyse der Mischung ergab folgendes Verhältnis: *N*-(3',4',5'-Trifluor-5-fluorbiphenyl)acetamid : 3,3',4,4',5,5'-Hexafluorobiphenyl : *N*-(2-Brom-4-fluorphenyl)acetamid = 96,5 : 3,5 : 0.

### Beispiel 8: Kupplung von N-(2-Brom-4-fluorphenyl)acetamid mit Natriumtetrakis(3,4,5-trifluorphenyl)borat in Gegenwart von 1,1-Bis-(di-tert-butylphosphino)ferrocen

106,1 mg [457 µmol] *N*-(2-Brom-4-fluorphenyl)acetamid, 66,6 mg [119 µmol] Natriumtetrakis-(3,4,5-trifluorphenyl)borat und 87,0 mg [821 µmol] Natriumcarbonat werden unter Ausschluss von Sauerstoff in 0,8 ml Toluol und 0,2 ml Wasser vorgelegt. Zu dieser Mischung werden 8,02 mg [16,9 µmol] 1,1-Bis-(di-tert-butylphosphino)ferrocen (Maßlösung in Toluol) und 13 mg [14,2 µmol] Pd₂dba₃ zugesetzt. Die Reaktionsmischung wird 65 Stunden bei 82 °C gerührt. Nach Beendigung der Umsetzung (HPLC-Kontrolle) wird die Reaktionsmischung auf RT abgekühlt, 2 ml Acetonitril zugefügt und anschließend über einen Nylon-Filter (Porengröße 0,45 µm) filtriert. Die HPLC-Analyse der Mischung ergab folgendes Verhältnis: *N*-(3',4',5'-Trifluor-5-fluorbiphenyl)acetamid : 3,3',4,4',5,5'-Hexafluorobiphenyl : *N*-(2-Brom-4-fluorphenyl)acetamid = 99,0 : 1,0 : 0.

### Beispiel 9: Kupplung von N-(2-Brom-4-fluorphenyl)acetamid mit Natriumtetrakis(3,4,5-trifluorphenyl)borat in Gegenwart von 1,2,3,4,5-Pentaphenyl-1'-(di-tert-butylphosphino)-ferrocen

102,7 mg [443 µmol] *N*-(2-Brom-4-fluorphenyl)acetamid, 65,8 mg [118 µmol] Natriumtetrakis-(3,4,5-trifluorphenyl)borat und 92,2 mg [870 µmol] Natriumcarbonat werden unter Ausschluss von Sauerstoff in 0,8 ml Toluol und 0,2 ml Wasser vorgelegt. Zu dieser Mischung werden 11,95 mg [16,8 µmol] 1,2,3,4,5-Pentaphenyl-1'-(di-tert-butylphosphino)ferrocen (Maßlösung in Toluol) und 8,1 mg [8,9 µmol] Pd₂dba₃ zugesetzt. Die Reaktionsmischung wird 65 Stunden bei 82 °C gerührt. Nach Beendigung der Umsetzung (HPLC-Kontrolle) wird die Reaktionsmischung auf RT abgekühlt, 2 ml Acetonitril zugefügt und anschließend über einen Nylon-Filter (Porengröße 0,45 µm) filtriert. Die HPLC-Analyse der Mischung ergab folgendes Verhältnis: *N*-(3',4',5'-Trifluor-5-fluorbiphenyl)acetamid : 3,3',4,4',5,5'-Hexafluorobiphenyl : *N*-(2-Brom-4-fluorphenyl)acetamid = 98,5 : 1,5 0.

### Beispiel 10: Kupplung von N-(2-Brem-4-fluorphenyl)acetamid mit Natriumtetrakis(3,4,5-trifluorphenyl)borat in Gegenwart von Tri(tert-butyl)phosphin

5 g [21,55 mmol] *N*-(2-Brom-4-fluorphenyl)acetamid, 3,25 g [5,82 mmol] Natriumtetrakis-(3,4,5-trifluorphenyl)borat und 4,57 g [43,1 mol] Natriumcarbonat werden unter Ausschluss von Sauerstoff in 30 ml Toluol und 10 ml Wasser vorgelegt. Zu dieser Mischung wird eine Lösung aus 348 mg [1,724 mmol] Tri(tert-butyl)phosphin und 786 mg [0,858 mmol] Pd₂dba₃ in 10 ml Toluol zugesetzt. Die Reaktionsmischung wird 24 Stunden bei 82°C gerührt. Die Reaktionsmischung wird auf RT abgekühlt und anschließend mit 70 ml Toluol und 50 ml Wasser versetzt. Die organische Phase wird über Celite abfiltriert und einrotiert. Man erhält 5,83 g *N*-(3',4',5'-Trifluor-5-fluorbiphenyl)acetamid mit einer GC-Reinheit von 69,8%.

### Beispiel 11: Herstellung von Natriumtetrakis(3,4,5-trifluorphenyl)borat

Zu einer Suspension aus 4,15 g [171 mmol] Magnesium und 4,74 g [43 mmol] Natriumtetrafluorborat in 50 ml Diethylether werden unter Ausschluss von Sauerstoff 35 g [166 mmol] 5-Brom-1,2,3-trifluorbenzol in 100 ml Diethylether in ca. 2 Stunden unter leichtem Rückfluss zugetropft. Der Grignard start nach ca. 5%-Zugabe von 5-Brom-1,2,3-trifluorbenzol. Die Reaktionsmischung wird 12 Stunden bei Raumtemperatur nachgerührt. Man lässt das Gemisch zu einer Lösung aus 50 g Natriumcarbonat in 700 ml Wasser fliessen. Nach dem Abdestillieren von Dimethylether wird die wässrige Phase mit Methyltertbutylether extrahiert, die vereinigten organischen Phasen werden mit Na₂SO₄ getrocknet und das Lösungsmittel im Vakuum entfernt. Nach Waschen mit wenig Wasser und Trocknen erhielt man Natriumtetrakis(3,4,5-trifluorphenyl)borat. ¹H NMR (CD₃CN) δ 6,80-6,71 (m, 8H); ¹³C NMR 8 157,4; 150,8; 137,0; 118,1.

## Patentansprüche

1. Verfahren zur Herstellung von substituierten Biphenylen der Formel (I) 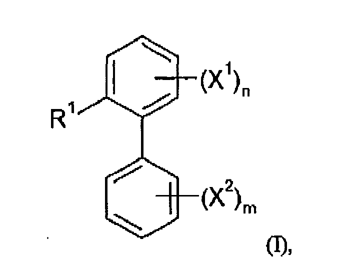 wobei
X¹ ausgewählt ist aus Halogenatomen und linearen oder verzweigten C₁₋₁₂-AlkylGruppen;
X² ausgewählt ist aus Halogenatomen;
n 0,1 oder 2 ist;
m 1, 2, 3, 4 oder 5 ist;
R¹ ausgewählt ist aus der Gruppe bestehend aus Amino- (NHR²), Nitro- (NO₂), Amid-Gruppen (R²-(CO)-NH-) oder Schiffschen Basen (R³R⁴C=N-),
R², R³ und R⁴ ausgewählt sind aus linearen oder verzweigten C₁₋₁₂-Alkyl-Gruppen oder cyclischen C₃₋₈-Alkyl-Gruppen, Benzyl-Gruppen, Benzoyl-Gruppen, Pyrazolyl-Gruppen der Formel (Ia), Pyridyl-Gruppen der Formel (Ib) 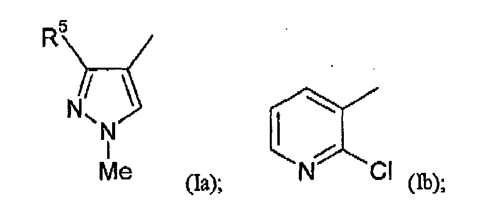
R⁵ eine lineare oder verzweigte C₁₋₁₂-Alkyl-Gruppe, oder eine C₁₋₆-Haloalkyl-Gruppe mit 1 bis 6 Halogenatomen ist;
durch Umsetzung von Arylhalogeniden der Formel (II) 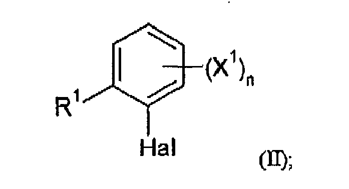 wobei
Hal ausgewählt ist aus Brom, Chlor und Iod,
R¹, X¹ und n den obigen Definitionen entsprechen;
in Gegenwart einer Base und eines Palladiumkatalysators in einem Lösungsmittel,
mit Tetraarylboraten der Formel (III) 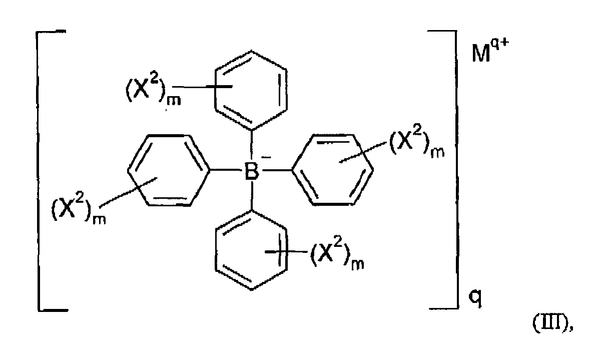 wobei
X² und m den obigen Definitionen entsprechen und
M^{q+} ein Kation ist, das ausgewählt ist aus Ammonium-(q=l), Alkalimetall- (q=1) und Erdalkalimetall-Kationen (q=2).

2. Verfahren gemäß Anspruch 1, **dadurch gekennzeichnet, dass**
X¹ 5-Fluor ist;
X² 3/4-Chlor ist;
n 1 ist;
m 2 ist;
R¹ ausgewählt ist aus der Gruppe bestehend aus Amino- (NN₂), Nitro- (NO₂), Amid-Gruppen (R²-(CO)-NH-) oder Schiffschen Basen (R₃R⁴C=N-),
R², R³ und R⁴ ausgewählt sind aus linearen oder verzweigten C₁₋₁₂-Alkyl-Gruppen oder cyclischen C₃₋₈-Alkyl-Gruppen

3. Verfahren gemäß Anspruch 1, **dadurch gekennzeichnet, dass**
X¹ Wasserstoff ist;
X² 3,4,5-Fluor ist;
n 1 ist;
m 3 ist;
R¹ ausgewählt ist aus der Gruppe bestehend aus Amino- (NH₂), Nitro- (NO₂), Amid-Gruppen (R²-(CO)-NH-) oder Schiffschen Basen (R³R⁴C=N-),
R², R³ und R⁴ ausgewählt sind aus linearen oder verzweigten G₁₋₁₂-Alkyl-Gruppen oder cyclischen C₃₋₈-Alkyl-Gruppen,

4. Verfahren gemäß Anspruch 1, wobei die Arylhalogenide der Formel (II) ausgewählt sind, aus der Gruppe bestehend aus N-(2-Brom-4-fluorophenyl)acetamid, N-(2-Chlor-4-fluorphenyl)-acetamid, N-(2-Bromphenyl)acetamid, N-(2-Chlorophenyl)acetamid, N-(2-Chlorphenyl)-3-oxobutanamid, N-(2-Bromophenyl)-3-oxobutanamid, N-(2-Ghlor-4-fluorphenyl)-3-oxobutanamid, N-(2-Brom-4-fluorphenyl)-3-oxobutanamid, 2-Brom-N-(propan-2-yliden)-anilin, 2-Chlor-N-(propan-2-yliden)anilin, 2-Brom-4-fluor-N-(propan-2-yliden)anilin, 2-chloro-4-fluoro-N-(propan-2-ylidene)anilin.

5. Verfahren gemäß einem der Ansprüche 1 bis 4, wobei das Tetraarylborat der Formel (III) ausgewählt ist aus der Gruppe bestehend aus Natriumtetrakis(3,4-dichlorphenyl)borat, Kaliumtetrakis(3,4-dichlorphenyl)borat, Natriumtetrakis(4-chlorphenyl)borat, Kaliumtetrakis(4-chlorphenyl)borat, Natriumtetrakis(3,4,5-trifluorphenyl)borat, Kaliumtetrakis(3,4,5-trifluorphenyl)borat.

6. Verfahren gemäß einem der Ansprüche 1 bis 5, wobei der Palladiumkatalysator ausgewählt ist aus
a) Palladiuin-Komplexe umfassend Palladium in der Oxidationssufe Null und Phosphinliganden der allgemeinen Formel PR'₃, wobei R' unabhängig voneinander ausgewählt ist aus der Gruppe bestehend aus C₁₋₆-Alkyl-, C₃₋₅-Cycloalkyl und C₆₋₁₂-Aryl oder Phosphinoferrocenliganden;
b) Palladiumsalzen in Gegenwart von Phosphinliganden der allgemeinen Formel PR'₃, wobei R' unabhängig voneinander ausgewählt ist aus der Gruppe bestehend aus C₁₋₆-Alkyl-, C₃₋₅-Cycloalkyl und C₆₋₁₂-Aryl oder in Gegenwart von Phosphinoferrocenliganden;
c) Palladiummetall, das ggf. auf einem Träger aufgebracht ist, wobei optional Phosphinliganden der allgemeinen Formel PR'₃, wobei R' unabhängig voneinander ausgewählt ist aus der Gruppe bestehend aus C₁₋₆-Alkyl-, C₃₋₅-Cycloalkyl und C₆₋₁₂-Aryl oder Phosphinoferrocenliganden zugegeben werden können.

7. Verfahren gemäß einem der Ansprüche 1 bis 6, wobei der Palladiumkatalysator der Kategorie (a) ausgewählt ist aus der Gruppe bestehend aus Tetrakis(triphenylphosphin)palladium, Tetrakis(tri-tert-botylphosphin)palladium, Adamantan-1-yl(adamantan-2-yl)butylphosphinpalladium, Biphenyl-2-yl(di-tert-butyl)phosphinpalladium oder 1,1-Bis-(di-tert-butylphosphino)ferrocenpalladium, Pentaphenyl(di-tert-butylphosphino)ferrocenpalladium, 1,3-Bis-(di-tert-butylphosphinomethylen)phenylpalladium.

8. Verfahren gemäß einem der Ansprüche 1 bis 7, wobei der Palladium Katalysator der Kategorie (b) ausgewählt ist aus der Gruppe bestehend aus Palladiumchlorid, Palladiumacetat oder Bisacetonitrilpalladiumchlorid, Palladium(II)dibenzylidenaceton, Bisacetylacetonatpalladium.

9. Verfahren gemäß einem der Ansprüche 1 bis 8, wobei 0.001 bis 10.0 mol% des Palladium Katalysators - bezogen auf das Arylhalogenid der Formel (II) - eingesetzt werden.

10. Verfahren gemäß einem der Ansprüche 1 bis 9, **dadurch gekennzeichnet, dass** die Umsetzung bei einer Temperatur von 20 bis 100°C erfolgt.

11. Verfahren gemäß einem der Ansprüche 1 bis 10, wobei das Lösungsmittel ein Gemisch aus Wasser und wenigstens einem organischen Lösungsmittel ist.

12. Verfahren gemäß Anspruch 11, wobei das organische Lösungsmittel Toluol ist.

## Claims

1. Process for the preparation of substituted biphenyls of the formula (I) 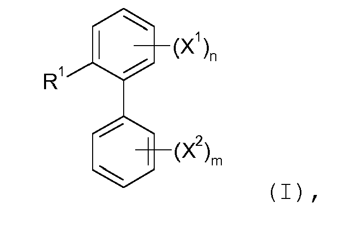 in which
X¹ is selected from halogen atoms and linear or branched C₁₋₁₂-alkyl groups;
X² is selected from halogen atoms;
n is 0, 1 or 2;
m is 1, 2, 3, 4 or 5;
R¹ is selected from the group consisting of amino (NHR²), nitro (NO₂), amide groups (R²-(CO)-NH-) or Schiff's bases (R³R⁴C=N-),
R², R³ and R⁴ are selected from linear or branched C₁-₁₂-alkyl groups or cyclic C₃₋₈-alkyl groups, benzyl groups, benzoyl groups, pyrazolyl groups of the formula (Ia), pyridyl groups of the formula (Ib) 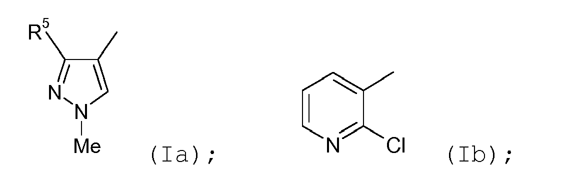
R⁵ is a linear or branched C₁₋₁₂-alkyl group or a C₁₋₆-haloalkyl group having 1 to 6 halogen atoms;
by reacting aryl halides of the formula (II) 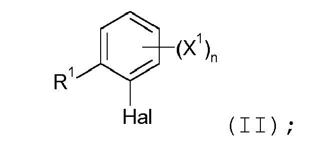 in which
Hal is selected from bromine, chlorine and iodine,
R¹, X¹ and n correspond to the above definitions;
in the presence of a base and of a palladium catalyst in a solvent,
with tetraarylborates of the formula (III) 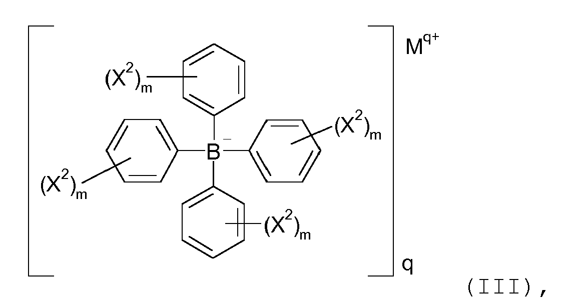 in which
X² and m correspond to the above definitions and
M^{q+} is a cation which is selected from ammonium (q=1), alkali metal (q=1) and alkaline earth metal cations (q=2).

2. Process according to Claim 1, **characterized in that**
X¹ is 5-fluoro;
X² is 3/4-chloro;
n is 1;
m is 2;
R¹ is selected from the group consisting of amino (NH₂), nitro (NO₂), amide groups (R²-(CO)-NH-) or Schiff's bases (R³R⁴C=N-),
R², R³ and R⁴ are selected from linear or branched C₁₋₁₂-alkyl groups or cyclic C₃₋₈-alkyl groups.

3. Process according to Claim 1, **characterized in that**
X¹ is hydrogen;
X² is 3,4,5-fluoro;
n is 1;
m is 3;
R¹ is selected from the group consisting of amino (NH₂), nitro (NO₂), amide groups (R²-(CO)-NH-) or Schiff's bases (R³R⁴C=N-),
R², R³ and R⁴ are selected from linear or branched C₁-₁₂-alkyl groups or cyclic C₃₋₈-alkyl groups.

4. Process according to Claim 1, the aryl halides of the formula (II) being selected from the group consisting of N-(2-bromo-4-fluorophenyl)acetamide, N-(2-chloro-4-fluorophenyl)acetamide, N-(2-bromophenyl)acetamide, N-(2-chlorophenyl)-acetamide, N-(2-chlorophenyl)-3-oxobutanamide, N-(2-bromophenyl)-3-oxobutanamide, N-(2-chloro-4-fluorophenyl)-3-oxobutanamide, N-(2-bromo-4-fluorophenyl)-3-oxobutanamide, 2-bromo-N-(prop-2-ylidene)aniline, 2-chloro-N-(prop-2-ylidene)aniline, 2-bromo-4-fluoro-N-(prop-2-ylidene)aniline, 2-chloro-4-fluoro-N-(prop-2-ylidene)aniline.

5. Process according to any of Claims 1 to 4, the tetraarylborate of the formula (III) being selected from the group consisting of sodium tetrakis(3,4-dichlorophenyl)borate, potassium tetrakis(3,4-dichlorophenyl)borate, sodium tetrakis(4-chlorophenyl)borate, potassium tetrakis(4-chlorophenyl)borate, sodium tetrakis(3,4,5-trifluorophenyl)borate, potassium tetrakis(3,4,5-trifluorophenyl)borate.

6. Process according to any of Claims 1 to 5, the palladium catalyst being selected from
a) palladium complexes comprising palladium in the oxidation state zero and phosphine ligands of the general formula PR'₃, in which R', independently of one another, is selected from the group consisting of C₁₋₆-alkyl, C₃₋₅-cycloalkyl and C₆₋₁₂-aryl or phosphinoferrocene ligands;
b) palladium salts in the presence of phosphine ligands of the general formula PR'₃, in which R', independently of one another, is selected from the group consisting of C₁₋₆-alkyl, C₃₋₅-cycloalkyl and C₆₋₁₂-aryl or in the presence of phosphinoferrocene ligands;
c) palladium metal which is optionally applied to a support, it optionally being possible to add phosphine ligands of the general formula PR'₃, in which R', independently of one another, is selected from the group consisting of C₁₋₆-alkyl, C₃₋₅-cycloalkyl and C₆₋₁₂-aryl or phosphinoferrocene ligands.

7. Process according to any of Claims 1 to 6, the palladium catalyst of category (a) being selected from the group consisting of tetrakis(triphenylphosphine)palladium, tetrakis(tri-tert-butylphosphine)palladium, adamant-1-yl(adamant-2-yl)butylphosphinepalladium, biphenyl-2-yl(di-tert-butyl)phosphinepalladium or 1,1-bis(di-tert-butylphosphino)ferrocenepalladium, pentaphenyl(di-tert-butylphosphino)-ferrocenepalladium, 1,3-bis(di-tert-butylphosphinomethylene)phenylpalladium.

8. Process according to any of Claims 1 to 7, the palladium catalyst of category (b) being selected from the group consisting of palladium chloride, palladium acetate or bisacetonitrilepalladium chloride, palladium(II)dibenzylideneacetone, bisacetylacetonatepalladium.

9. Process according to any of Claims 1 to 8, 0.001 to 10.0 mol% of the palladium catalyst - based on the aryl halide of the formula (II) - being used.

10. Process according to any of Claims 1 to 9, **characterized in that** the reaction is effected at a temperature of 20 to 100°C.

11. Process according to any of Claims 1 to 10, the solvent being a mixture of water and at least one organic solvent.

12. Process according to Claim 11, the organic solvent being toluene.

## Revendications

1. Procédé pour la préparation de biphénylène substitué de formule (I) 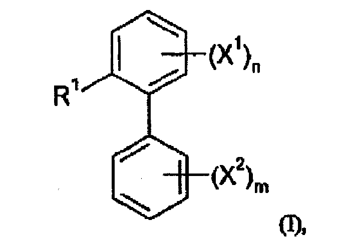 dans laquelle
X¹ est choisi parmi des atomes d'halogène et des groupes alkyle en C₁-C₁₂ linéaires ou ramifiés ;
X² est choisi parmi des atomes d'halogène ;
n est 0, 1 ou 2 ;
m est 1, 2, 3, 4 ou 5 ;
R¹ est choisi dans le groupe constitué par des groupes amino (NHR²), nitro (NO₂), amido (R²-(CO)-NH-) ou des bases de Schiff (R³R⁴C=N-),
R², R³ et R⁴ sont choisis parmi des groupes alkyle en C₁-C₁₂ linéaires ou ramifiés ou des groupes alkyle cycliques en C₃-C₈, des groupes benzyle, des groupes benzoyle, des groupes pyrazolyle de formule (Ia), des groupes pyridyle de formule (Ib) 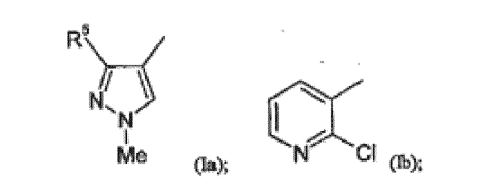
R⁵ est un groupe alkyle en C₁-C₁₂ linéaire ou ramifié, ou un groupe halogénoalkyle(C₁-C₆) comportant de 1 à 6 atomes d'halogène ;
par mise en réaction d'halogénures d'aryle de formule (II) 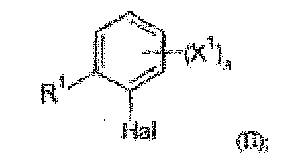 dans laquelle
Hal est choisi parmi le brome, le chlore et l'iode,
R¹, X¹ et n correspondent aux définitions ci-dessus ;
en présence d'une base et d'un catalyseur au palladium dans un solvant,
avec des tétraarylborates de formule (III) 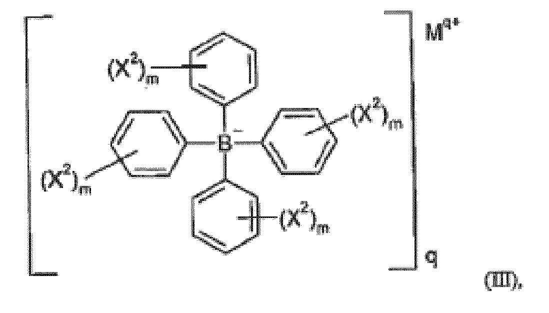 dans laquelle
X² et m correspondent aux définitions ci-dessus et
M^{q+} est un cation, qui est choisi parmi les cations ammonium (q = 1), de métaux alcalins (q = 1) et de métaux alcalino-terreux (q = 2).

2. Procédé selon la revendication 1, **caractérisé en ce que**
X¹ est 5-fluoro ;
X² est 3/4-chloro ;
n est 1 ;
m est 2 ;
R¹ est choisi dans le groupe constitué par les groupes amino (NH₂) , nitro (NO₂), amido (R²-(CO)-NH-) ou des bases de Schiff (R³R⁴C=N-),
R², R³ et R⁴ sont choisis parmi des groupes alkyle en C₁-C₁₂ linéaires ou ramifiés ou des groupes alkyle cycliques en C₃-C₈.

3. Procédé selon la revendication 1, **caractérisé en ce que**
X¹ est un atome d'hydrogène ;
X² est 3,4,5-fluoro ;
n est 1 ;
m est 3 ;
R¹ est choisi dans le groupe constitué par les groupes amino (NH₂), nitro (NO₂), amido (R²-(CO)-NH-) ou des bases de Schiff (R³R⁴C=N-),
R², R³ et R⁴ sont choisis parmi des groupes alkyle en C₁-C₁₂ linéaires ou ramifiés ou des groupes alkyle cycliques en C₃-C₈.

4. Procédé selon la revendication 1, dans lequel les halogénures d'aryle de formule (II) sont choisis dans le groupe constitué par le N-(2-bromo-4-fluorophényl)acétamide, le N-(2-chloro-4-fluorophényl)-acétamide, le N-(2-bromophényl)acétamide, le N-(2-chlorophényl)acétamide, le N-(2-chlorophényl)-3-oxobutanamide, le N-(2-bromophényl)-3-oxobutanamide, le N-(2-chloro-4-fluorophényl)-3-oxobutanamide, le N-(2-bromo-4-fluorophényl)-3-oxobutanamide, la 2-bromo-N-(propan-2-ylidène)-aniline, la 2-chloro-N-(propan-2-ylidène)aniline, la 2-bromo-4-fluor-N-(propan-2-ylidène)aniline, la 2-chloro-4-fluoro-N-(propan-2-ylidène)aniline.

5. Procédé selon l'une quelconque des revendications 1 à 4, dans lequel le tétraarylborate de formule (III) est choisi dans le groupe constitué par le tétrakis(3,4-dichlorophényl)borate de sodium, le tétrakis(3,4-dichlorophényl)borate de potassium, le tétrakis(4-chlorophényl)borate de sodium, le tétrakis(4-chlorophényl)borate de potassium, le tétrakis(3,4,5-trifluorophényl)borate de sodium, le tétrakis(3,4,5-trifluorophényl)borate de potassium.

6. Procédé selon l'une quelconque des revendications 1 à 5, dans lequel le catalyseur au palladium est choisi parmi
a) des complexes de palladium comprenant du palladium au degré d'oxydation zéro et des ligands phosphine de formule générale PR'₃, dans laquelle R' est choisi chaque fois indépendamment dans le groupe constitué par des ligands alkyle en C₁-C₆, cycloalkyle en C₃-C₅ et aryle en C₆-C₁₂ ou des ligands phosphinoferrocène ;
b) des sels de palladium en présence de ligands phosphine de formule générale PR'₃, dans laquelle R' est choisi chaque fois indépendamment dans le groupe constitué par des ligands alkyle en C₁-C₆, cycloalkyle en C₃-C₅ et aryle en C₆-C₁₂ ou en présence de ligands phosphinoferrocène ;
c) le palladium métallique, qui est éventuellement appliqué sur un support, en option pouvant être ajoutés des ligands phosphine de formule générale PR'₃, dans laquelle R' est choisi chaque fois indépendamment dans le groupe constitué par des ligands alkyle en C₁-C₆, cycloalkyle en C₃-C₅ et aryle en C₆-C₁₂ ou des ligands phosphinoferrocène.

7. Procédé selon l'une quelconque des revendications 1 à 6, dans lequel le catalyseur au palladium de la catégorie (a) est choisi dans le groupe constitué par le tétrakis(triphénylphosphine)palladium, le tétrakis(tri-tert-butylphosphine)palladium, l'adamantan-1-yl(adamantan-2-yl)butylphosphine-palladium, le biphényl-2-yl(di-tert-butyl)phosphine-palladium ou le 1,1-bis-(di-tert-butylphosphino)-ferrocènepalladium, le pentaphényl(di-tert-butyl-phosphino)ferrocènepalladium, le 1,3-bis-(di-tert-butylphosphinométhylène)phénylpalladium.

8. Procédé selon l'une quelconque des revendications 1 à 7, dans lequel le catalyseur au palladium de la catégorie (b) est choisi dans le groupe constitué par le chlorure de palladium, l'acétate de palladium ou le chlorure de bisacétonitrilepalladium, la palladium(II)dibenzylidène-acétone, le bisacétylacétonatepalladium.

9. Procédé selon l'une quelconque des revendications 1 à 8, dans lequel on utilise de 0,001 à 10,0 % en moles du catalyseur au palladium - par rapport à l'halogénure d'aryle de formule (II).

10. Procédé selon l'une quelconque des revendications 1 à 9, **caractérisé en ce que** la réaction s'effectue à une température de 20 à 100 °C.

11. Procédé selon l'une quelconque des revendications 1 à 10, dans lequel le solvant est un mélange d'eau et d'au moins un solvant organique.

12. Procédé selon la revendication 11, dans lequel le solvant organique est le toluène.
